# EUROPEAN PATENT APPLICATION

(11) **EP 3 772 357 A1**
(43) Date of publication of application: **10.02.2021**
(21) Application number: 20190098.2
(22) Date of filing: 07.08.2020
(51) Int. Cl.: A61M 1/00, A61B 17/22

(54) **THROMBECTOMY WITH VENTURI SUCTION**

(30) Priority: 08.08.2019 US 201916535947
(71) Applicant: DePuy Synthes Products, Inc., Raynham, MA 02767 (US)
(72) Inventor: LORENZO, Juan, DAVIE, FL Florida 33328 (US)
(74) Representative: Small, Gary James

(57) **Abstract**

A venturi pump can provide suction through a catheter during an intravascular treatment. The venturi pump can have a supply port, exhaust port, and vacuum inlet such that a fluidic flow from the supply port to the exhaust port produces suction at the vacuum inlet due to the Venturi effect. A device or assembly can include the venturi pump, a flow regulator, and a sealable entrance sized to receive the catheter. The flow regulator can be manipulated to adjust suction through the vacuum inlet of the venturi pump. The device or assembly can have an exit and a passageway between the entrance and the exit to allow a pull wire, Intermediate Catheter, or other clot retrieval system elongated member to pass therethrough and be manipulated during the intravascular treatment.

## Description

### Field of Invention

The present invention generally relates to intravascular medical treatments, and more particularly, to regulating aspirated blood flow rate during a thrombectomy procedure.

### Background

During intravascular medical treatments it can be advantageous to provide suction at a treatment site within a patient. During a thrombectomy, for example, a physician can utilize a syringe or a vacuum pump to produce suction in the vicinity of the thrombus (clot) and either extract the thrombus solely via suction (e.g. via A Direct Aspiration first-Pass Thrombectomy (ADAPT) technique) or use suction in conjunction with a clot retriever device. The syringe or vacuum pump can be connected to the proximal end of the Intermediate or Guide Catheter (e.g. Balloon Guide Catheter) and the vacuum can communicate with the distal tip of the catheter through the lumen of the catheter.

Generally, thrombectomy suction sources currently available include electrical pumps such as the Penumbra Max, hand operated mechanical pumps such as the ASPIRE aspiration device (Control Medical Technology, LLC), and locking syringes such as the 60-mL Medallion (Merit Medical, Inc.). Electrical pumps typically include a collection canister and a control knob for controlling vacuum pressure. Hand operated mechanical pumps typically utilize a barrel and plunger to create a vacuum when manipulated by hand and a one-way valve to purge the barrel. Locking syringes typically utilize a barrel and a plunger that can be locked in place.

Some factors to consider when selecting a suction source for the thrombectomy include the ability to maintain suction during the course of the procedure, the ease of operation during the procedure, maintenance of the equipment between procedures, and expense. Compared to an electrical or mechanical pump, a locking syringe is generally easier to use during the procedure because it can be mounted directly to a side port of a Rotating Hemostasis Valve (RHV), it's inexpensive, and it's disposable, therefore low maintenance. However, the locking syringe cannot be purged without losing suction, and therefore has the potential to fill with blood and lose suction before the procedure is completed. Although both the electrical pumps and mechanical pumps can be purged, they generally are larger and therefore more difficult to manipulate during a procedure, more expensive, and more difficult to maintain.

There is therefore a need for improved methods, devices, and systems for controlling suction during thrombectomy procedures. Similarly, control of suction is potentially beneficial in other intravascular or medical treatments where an aspiration pump or suction syringe is used.

### Summary

It is an object of the present invention to provide systems, assemblies, devices, and methods to meet the above-stated needs. Generally, it is an object of the present invention to provide devices and methods that utilize a venturi pump to provide suction through a catheter as part of a thrombectomy.

An example suction device can include an entrance with a movable seal for sealing around an outer diameter of a catheter, a venturi pump having a supply port, an exhaust port, and a vacuum inlet, and a portable housing providing structural support for the entrance and the venturi pump. The vacuum inlet of the venturi pump can be in fluidic communication with the entrance of the suction device such that the venturi pump can provide suction to a lumen of the catheter when the catheter is sealed by the seal the entrance. The supply port can receive a positive pressure gas supply sufficient to generate suction at the vacuum inlet of the venturi pump.

The suction device can also include a flow regulator valve connected to the supply port of the venturi pump to regulate fluidic flow into the supply port.

The suction device can also include a compressed gas canister that provides the positive pressure gas supply at the supply port of the venturi pump. The canister can be detachably attached to the supply port. The canister can be structurally supported by the portable housing.

The suction device can also include a discharge container. The discharge container can be positioned to receive discharge from the exhaust port of the venturi pump.

The suction device can also include an exit having a movable seal that can seal around an outer diameter of a pull wire, Intermediate Catheter, or other inner elongated member positioned within the catheter. The seal at the exit can hemostatically seal around the inner elongated member. The exit can be structurally supported by the portable housing.

The portable housing can have a passageway that is sized to allow the inner elongated member to extend between the entrance and the exit. The passageway can also be sized to provide a flow path between the entrance and the vacuum inlet when the elongated member is extended between the entrance and exit.

The entrance and exit of the suction device can each include locking mechanisms to inhibit longitudinal and rotational movement of the catheter and inner elongated member respectively.

In some applications, the catheter can be a Guide Catheter and the inner elongated member can be an Intermediate Catheter. The Guide Catheter can be a Balloon Guide Catheter providing aspiration during a thrombectomy, and the Intermediate Catheter can deliver a clot capture device such as a stentriever. The passageway can be sized to allow an Intermediate Catheter having an outer diameter of about 2 mm to extend between the entrance and exit while also providing a flow path between the entrance and the vacuum inlet of the venturi pump.

In other applications, the catheter can be suitable for capturing a clot as part of A Direct Aspiration first-Pass Technique. In such applications, suction can be provided via the venturi pump that is sufficient for capturing the clot and securing the clot to the catheter as the clot and catheter are extracted from the patient. The suction can be generated by flowing gas from the positive pressure supply from the supply port to the exhaust port, generating a vacuum at the vacuum inlet as a result of the gas flow, generating suction through a fluidic communication between the catheter lumen positioned at the entrance and the vacuum inlet as with the generated vacuum.

An example handheld endovascular treatment assembly can include a first, second, and third opening, and a venturi channel. The venturi channel can be in fluidic communication with the first, second, and third opening. The venturi channel can provide a restricted flow path from the second opening to the third opening and a vacuum inlet at the third opening.

The handheld assembly can include a flow regulator in communication with the venturi channel. The flow regulator can be used to adjust flow through the restricted flow path of the venturi channel by regulating flow into the second opening or the third opening.

The handheld assembly can include a compressed gas canister. The canister can be detachably attached within the assembly. The canister can be structurally supported within the assembly such that the canister is handheld at least by virtue of being part of the handheld assembly. The canister can provide fluidic flow into the second opening. The fluidic flow can be an gas flow.

The handheld assembly can include a fourth opening. The fourth opening can include a seal that can receive and hemostatically seal around an inner elongated member extending through the catheter. The assembly can include a passageway sized to allow the inner elongated member to extend between the first and fourth openings. The passageway can be sized to provide a flow path from the first opening to the third opening. The passageway can be sealed during a thrombectomy. The passageway can thereby both receive the inner elongated member and allow suction to be provided to the lumen of the catheter when the catheter is positioned in the first opening and when fluidic flow to the venturi channel is provided at the second opening. The passageway can receive an inner elongated member having an outer diameter of about 2 mm extending between the first and fourth opening while providing the flow path from the first opening to the third opening.

The first and fourth openings can each respectively having a locking mechanism to inhibit the catheter and inner elongated member respectively from moving rotationally and longitudinally.

The handheld assembly can be part of a system that includes the catheter. The catheter can be suitable for performing a thrombectomy using ADAPT.

An example method for performing a thrombectomy can include some or all of the following steps. Steps can be performed in various orders as would be understood by a person of ordinary skill in the art. Additional steps not listed here can be performed as part of the example method as would be understood by a person of ordinary skill in the art.

A catheter having a lumen therethrough can be selected. A venturi pump having a supply port, an exhaust port, and a vacuum inlet can be selected. A sealed flow path from the lumen of the catheter to the vacuum inlet of the venturi pump can be created. The venturi pump and the sealed flow path can be structurally supported by a portable housing.

Compressed gas can be flowed from the supply port to the exhaust port of the venturi pump, thereby creating suction at the vacuum inlet and through the sealed flow path. The suction can be provided through the lumen of the catheter to aspirate a blood vessel in the vicinity of a thrombus.

The portable housing can be held by hand the suction is provided through the lumen of the catheter to aspirate the blood vessel in the vicinity of the thrombus. The Intermediate Catheter can be manipulated while providing the suction through the lumen of the catheter to aspirate the blood vessel in the vicinity of the thrombus.

Compressed gas can be continuously flowed from the supply port to the exhaust port of the venturi pump to secure the thrombus to the catheter while the catheter is retracted proximally through the blood vessel. The thrombus can be extracted from the blood vessel with the catheter using ADAPT.

As an alternative to ADAPT, the thrombus can be extracted using an Intermediate Catheter for delivering a clot capture device. A proximal end of the catheter can be inserted through an entrance in the portable housing. The Intermediate Catheter can be extended from within the lumen of the catheter, out of the proximal end of the catheter, through the portable housing, and out an exit port in the portable housing. The outer diameter of the catheter can be sealed at the entrance of the portable housing. An outer diameter of the Intermediate Catheter can be sealed at the exit of the portable housing.

### Brief Description of the Drawings

The above and further aspects of this invention are further discussed with reference to the
following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
FIG. 1 is an illustration of an example system incorporating a venturi pump according to aspects of the present invention;
FIG. 2 is an illustration of another example system incorporating a venturi pump according to aspects of the present invention;
FIG. 3 is an illustration of another example system incorporating a venturi pump according to aspects of the present invention;
FIGs. 4A and 4B each illustrate a respective venturi pump according to aspects of the present invention; and
FIG. 5 is a flow diagram illustrating method steps for performing a thrombectomy according to aspects of the present invention.

### Detailed Description

Generally, it is an object of the present invention to provide systems, assemblies, devices, and methods that utilize a venturi pump to provide suction through a catheter during a thrombectomy. The venturi pump can have a supply port, exhaust port, and vacuum inlet such that a fluidic flow from the supply port to the exhaust port produces suction at the vacuum inlet due to the Venturi effect. The venturi pump can be attached to, or integrated with, one or more devices having a sealable entrance sized to receive the catheter. The venturi pump can be attached to, or integrated with, a flow regulator such that the flow regulator can be manipulated to adjust suction through the vacuum inlet of the venturi pump.

An assembly or device can include a "Rotating Hemostatis Valve" (RHV) connected to, or integrated with an aspiration control valve controllable by a switch, button, slider, trigger, grip, level , rotating wheel, rotating valve, handle or other interface that is conveniently positioned and configured to be manipulated while simultaneously stabilizing the RHV and catheter in one hand and/or retracting an inner elongated member with the other hand such as described in U.S. Patent Application Number 16/400,221 filed on May 1, 2019, currently pending, incorporated herein by reference. The RHV with the integrated or attached aspiration control valve can include the sealable entrance for receiving the catheter and the flow regulator. The venturi pump can be attached to or integrated with the RHV with the integrated aspiration control valve such that suction from the venturi pump is controllable by the integrated aspiration control valve and the RHV provides a flow path from the lumen of the catheter when positioned at the entrance of the RHV to the vacuum inlet of the venturi pump.

When the catheter is sealed at the entrance, an assembly or device including the venturi pump and entrance can include a flow path from the lumen of the catheter to the vacuum inlet. Flow into the vacuum inlet can be combined with flow from the supply port and exit the exhaust port. The flow path from the vacuum inlet to the supply port can be dimensioned such that the path is unlikely to become clogged as blood, liberated thrombus, and other obstructions originating from the blood vessel flow therethrough. A compressed gas canister can be connected to the supply port to provide fluidic flow through the venturi pump sufficient to create suction at the vacuum inlet. An assembly or device including the compressed gas canister, venturi pump, flow regulator, and entrance can be designed to be held by hand by a physician during a thrombectomy procedure.

Specific examples are illustrated in the figures. It is contemplated that one or more elements from a given example can be combined with one or more elements of another example and/or or with one or more known elements as would be understood by a person of ordinary skill in the art according to the present invention.

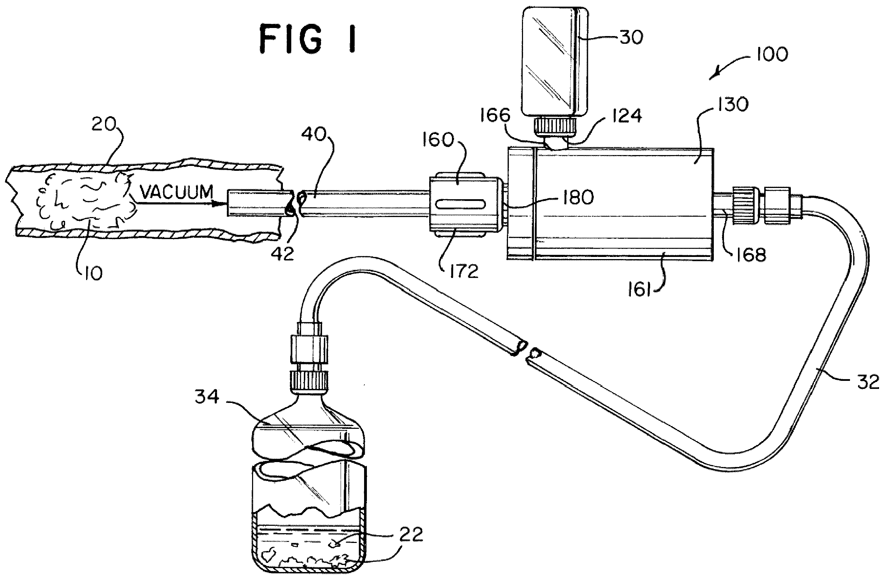

FIG. 1 is an illustration of an example of a system 100 that can be used to capture an obstruction such as a clot 10 using an aspiration device or assembly 160 with a venturi pump 130. The aspiration device or assembly 160 can include a venturi pump 130 having a supply port 166, vacuum inlet 180, and exhaust port 168. A luer lock 172 can be positioned in communication with the vacuum inlet 180. The luer lock 172 and venturi pump 130 can be separable components, or integrated. In either case, when the luer lock 172 and venturi pump 130 are connected, they can form an aspiration device 160 with an entrance 162 sized to receive a catheter 40, a supply port 166 that can receive a positive pressure gas supply, and an exhaust port 168 that can provide an outlet for gas and waste 22 extracted through the catheter 40. If the luer lock 172 and venturi pump 130 are separable components, the venturi pump can have a housing 161 that is portable and can provide structural support for the luer lock 172 and thereby the entrance 162 such that when assembled, the venturi pump 130 and luer lock 172 assembly is portable. The luer lock 172 and venturi pump 130 can alternatively be integrated with a common housing 161 providing structural support for the entrance 162.

A flow regulator 124 can be a separable component that can be attached to the venturi pump 130 at the supply port 166 of the venturi pump 130 to control flow into the supply port 166, or the flow regulator 124 can be integrated with the housing 161 to control flow downstream of the supply port 166. In either case, when connected to the venturi pump 130, the flow regulator 124 can be considered a component of the aspiration device 160.

The entrance 162 in the housing can include a seal such as a rotating hemostatic seal that can be opened with the rotating luer lock 172 to allow insertion of the catheter 40 and closed by rotating the luer lock 172 to seal the seal around the outer circumference of the catheter. The catheter 40 can be positioned within a blood vessel 20 of a patient such that the distal end of the catheter 40 is near the clot 10.

The flow regulator 124 can be closed and a compressed gas source 30 can be connected upstream of the flow regulator 124. The compressed gas source can be a compressed gas canister 30. The canister 30 can be attached and detached to and from the aspiration device 160 directly at to the supply port 166 if the flow regulator 124 is integral to the aspiration device 160 housing 161, or the gas canister 30 can be attached and detached indirectly to and from the aspiration device 160 by virtue of being attached to the flow regulator 124 if the flow regulator is not integral to the aspiration device 160 housing 161. In either case, when attached, the canister 30 and flow regulator 124 can be structurally supported by the housing 161 of the aspiration control device 160. The assembled canister 30, flow regulator 124 and aspiration device 160 can be sized, weighted, and otherwise designed to be moved and manipulated by hand as needed by a physician during a thrombectomy procedure.

The exhaust port 168 of the aspiration device 160 can be connected to a discharge container 34. A discharge tube 32 can link the exhaust port 168 to the discharge container 34. The discharge tube 32 and discharge container 34 can be configured such that gas exhaust and waste 22 can exit the aspiration control device 160 in such a way that is non-obstructive to a physician manipulating the device 160 during a thrombectomy. The discharge tube 32 can be a flexible tube. In some applications, the discharge container 34 can be placed near the patient during the thrombectomy, and the tubing 32 can be sufficiently long enough to allow movement of the device 160 and short enough so as to not become tangled or otherwise become difficult to manage.

Once the catheter 40, air canister 30, flow regulator 124, and discharge canister 34 are configured as described, the flow regulator 124 can be opened. Once the flow regulator 124 is open, gas from the canister 30 can flow through the venturi pump in the aspiration device 160 and out the exhaust port. The flow can generate suction at the vacuum inlet of the venturi pump. Suction at the vacuum inlet can be transferred to the lumen 42 of the catheter via a flow path in the aspiration device housing 161 between the entrance 162 and the supply port 166. The suction through the catheter lumen 42 can be sufficient to aspirate blood within the blood vessel 20, and/or the suction through the catheter lumen 42 can be sufficient to secure the clot 10 to the distal end of the catheter 40.

In some procedures, a physician can desire to modify the suction through catheter 40 during the procedure to suit specific aspects of a clinical case. For example, during a thrombectomy, a physician might prefer a lower suction force or slower aspiration flow rate on initial clot dislodgement, then increase suction when the clot 10 is approaching the catheter 40, and then further increase suction to maximum when pulling the clot 10 into the catheter 40. Increasing suction during clot retrieval can reduce the chance of vessel 20 collapse due to negative pressure in the vasculature 20. In some examples presented herein, the flow regulator 124 can be manipulated by the physician during a thrombectomy to adjust suction through the lumen 42 of the catheter.

The flow regulator 124 can include a valve that can be manipulated to adjust the velocity of gas flow through a venturi channel in the venturi pump. Because suction at the vacuum inlet of the venturi pump is a function of flow rate through the venturi channel, adjusting the velocity of gas flow through the venturi channel thereby adjusts suction provided at the vacuum inlet of the venturi pump. Because the vacuum inlet is in fluidic communication with the entrance of the aspiration device housing, 161 to which the catheter 40 can attach, adjusting the flow regulator 124 can thereby adjust the suction through the catheter 40.

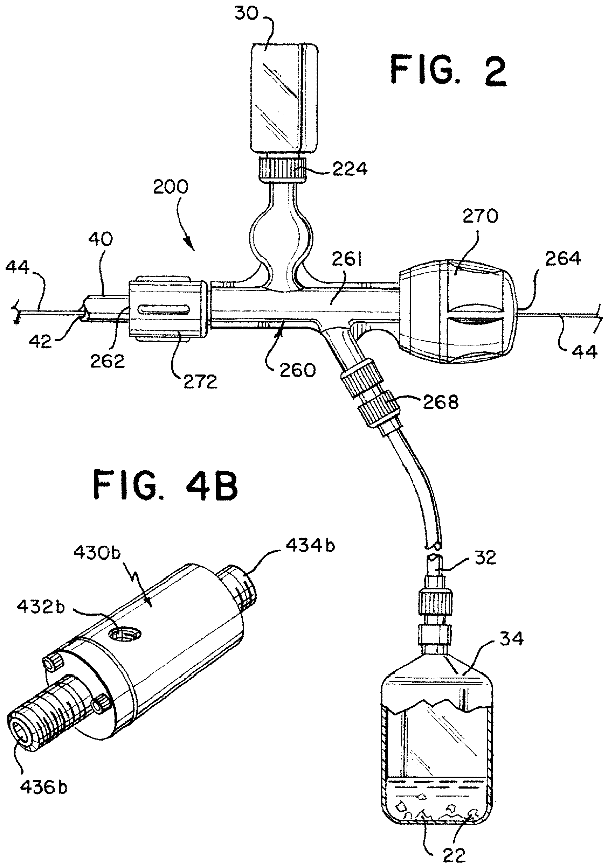

FIG. 2 illustrates another example system 200 that can be used to capture an obstruction such as a clot 10 using an aspiration device 260 with an integrated venturi pump. The aspiration device 260 can include a housing 261 having an entrance 262 sized to receive a catheter 40, a supply port 266 that can receive a positive pressure gas supply, an exhaust port 268 that can provide an outlet for gas and waste 22 extracted through the catheter 40, and an exit 264 sized to allow a pull wire, Intermediate Catheter, or other elongated member 44 to pass therethrough. A flow regulator 224 can be attached to the aspiration device 260 at the supply port 266 to control flow into the supply port 266, or the flow regulator 224 can be integrated with the housing 261 of the aspiration control device to control flow downstream of the supply port 266.

The aspiration device 260 can be shaped similar to a known RHV having an additional side port 268, or the aspiration device 260 can be shaped similar to an RHV with an integrated or attached aspiration control valve such as described in U.S. Patent Application Number 16/400,221. As illustrated in FIG. 2, an aspiration device 260 shaped as described can be modified to include the venturi pump integrated within the housing 261 of the device 260.

The entrance 262 in the housing 261 can include a seal such as a rotating hemostatic seal that can be opened with a rotating luer lock 272 to allow insertion of the catheter 40 and closed by rotating the luer lock 272 to seal the seal around the outer circumference of the catheter 40. The catheter 40 can be positioned within a blood vessel 20 of a patient such that the distal end of the catheter 40 is near the clot 10 such as illustrated in FIG. 1.

Referring to FIG. 2, the exit 264 in the housing 261 can include a seal such as a rotating hemostatic seal that can be opened with a rotating luer lock 270 to allow insertion of the inner elongated member 44 therethrough and to allow a physician to move the inner elongated member during a procedure. The seal can be closed with the rotating luer lock 270 to seal around the outer circumference of the inner elongated member 44.

The housing 261 can include a passageway that allows the inner elongated member 44 to extend between the exit 264 and the entrance 262 of the housing. The inner elongated member 44 can be positioned to extend within the lumen 42 of the catheter 40. The inner elongated member 44 can be a pull wire, Intermediate Catheter, part of an intralumenal device delivery system, etc. The passageway can be sized to allow the inner elongated member to pass therethrough while also providing the flow path between the catheter lumen 42 and the vacuum inlet of the integrated venturi pump. In some applications, the passageway can be sized to allow passage of an Intermediate Catheter having an outer diameter of about 2 mm while also providing the flow path between the catheter lumen 42 and the vacuum inlet of the venturi pump. When the entrance 262 is sealed around the catheter 40 and the exit 264 is sealed around the inner elongated member 44, the passageway can create a sealed flow path between the lumen 42 of the catheter 40 and the vacuum inlet of the venturi pump.

The flow regulator 224 can be closed and a compressed gas source 30 can be connected upstream of the flow regulator 224. The compressed gas source can be a compressed gas canister 30. The canister 30 can be attached and detached to and from the aspiration device 260 directly at to the supply port 266 if the flow regulator 224 is integral to the aspiration device housing 261, or the gas canister 30 can be attached and detached indirectly to and from the aspiration device 260 by virtue of being attached to the flow regulator 224 if the flow regulator is not integral to the aspiration device housing 261. In either case, when attached, the canister 30 and flow regulator 224 can be structurally supported by the housing 261 of the aspiration control device 260. The assembled canister 30, flow regulator 224 and aspiration device 260 can be sized, weighted, and otherwise designed to be moved and manipulated by hand as needed by a physician during a thrombectomy procedure.

The exhaust port 268 of the aspiration device 260 can be connected to a discharge container 34. A discharge tube 32 can link the exhaust port 268 to the discharge container 34. The discharge tube 32 and discharge container 34 can be configured such that gas exhaust and waste 22 can exit the aspiration control device 260 in such a way that is non-obstructive to a physician manipulating the device 260 during a thrombectomy. The discharge tube 32 can be a flexible tube. In some applications, the discharge container 34 can be placed near the patient during the thrombectomy, and the tubing 32 can be sufficiently long enough to allow movement of the device 260 and short enough so as to not become tangled or otherwise become difficult to manage.

Once the catheter 40, inner elongated member 44, air canister 30, flow regulator 224, and discharge canister 34 are configured as described, the flow regulator 224 can be opened. Once the flow regulator 224 is open, gas from the canister 30 can flow through the venturi pump in the aspiration device 260 and out the exhaust port 268. The flow can generate suction at the vacuum inlet of the venturi pump. Suction at the vacuum inlet can be transferred to the lumen 42 of the catheter via a flow path in the aspiration device housing 261 between the entrance 262 and the supply port 266. The suction through the catheter lumen 42 can be sufficient to aspirate blood within the blood vessel 20, and/or the suction through the catheter lumen 42 can be sufficient to secure the clot 10 to the distal end of the catheter 40.

The flow regulator 224 can be manipulated by the physician during a thrombectomy to adjust suction through the lumen 42 of the catheter. The flow regulator 224 can include a valve that can be manipulated to adjust the velocity of gas flow through a venturi channel in the venturi pump. Because suction at the vacuum inlet of the venturi pump is a function of flow rate through the venturi channel, adjusting the velocity of gas flow through the venturi channel thereby adjusts suction provided at the vacuum inlet of the venturi pump. Because the vacuum inlet is in fluidic communication with the entrance of the aspiration device housing, 261 to which the catheter 40 can attach, adjusting the flow regulator 224 can thereby adjust the suction through the catheter 40.

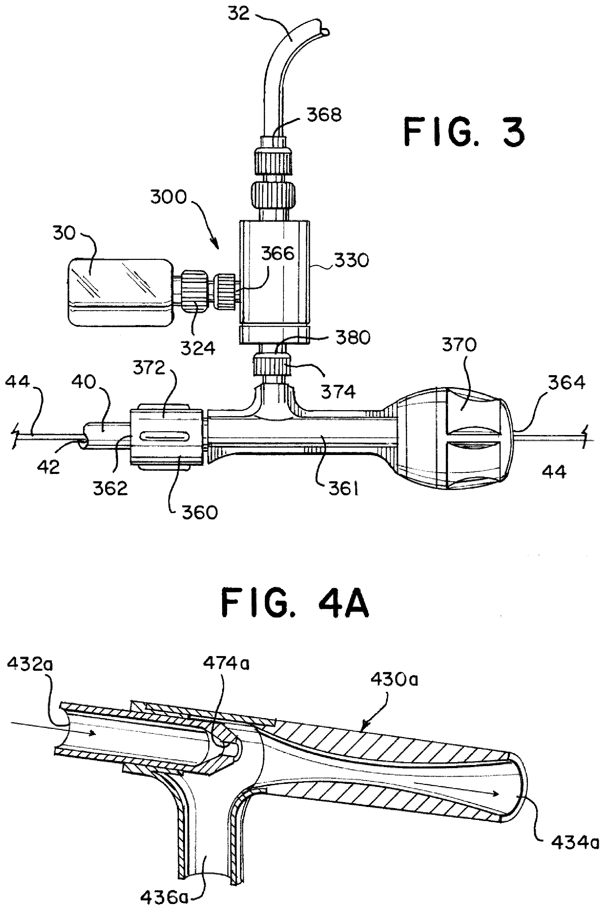

FIG. 3 illustrates an example system 300 that can be used to capture an obstruction such as a clot 10 using an assembly 360 having a venturi pump 330, a portable housing 361, and a flow regulator 324. The assembly 360 can be used to provide suction through a lumen 42 of a catheter 40 during a thrombectomy. The assembly 360 can receive an elongated member 44 extending within the catheter 40 such that the elongated member 44 can be manipulated during the thrombectomy to capture a clot at the distal end of the elongated member 44. Air supply 30 and exhaust 32 can be connected to the assembly 360 to provide suction through the lumen 42 of the catheter.

The venturi pump 330 can include a vacuum inlet 380, an exhaust port 368, and a supply port 366. The venturi pump 330 can be a separable component or can be integrated with the portable housing 361. In either case, the venturi pump 330 can be portable such that when the assembly 360 is assembled, the assembly 360 can be manipulated during a thrombectomy. The venturi pump 330 can be configured to provide a range of suction at the vacuum inlet 380 in response to adjusting a flow rate of gas into the supply port 366. The venturi pump 330 can include a passageway between the vacuum inlet 380 and the exhaust port 368 that is dimensioned such that the passageway is unlikely to clot as a result of waste from a blood vessel entering the vacuum inlet 380 of the venturi pump 330.

The housing 361 of the assembly 360 can include and/or support a side port 374 configured to receive a connection to a vacuum source, an entrance 362 sized to receive and seal a catheter 40, and an exit 364 sized receive and seal a pull wire, Intermediate Catheter, or other elongated member 44. The housing 361 can include a passageway that allows the inner elongated member 44 to extend between the exit 364 and the entrance 362 of the housing. The inner elongated member 44 can be positioned to extend within the lumen 42 of the catheter 40. The inner elongated member 44 can be a pull wire, Intermediate Catheter, part of an intralumenal device delivery system, etc. The passageway can be sized to allow the inner elongated member 44 to pass therethrough while also providing a flow path from the catheter lumen 42, through the side port 374, and into the vacuum inlet 380 of the attached venturi pump 330. In some applications, the passageway can be sized to allow passage of an Intermediate Catheter having an outer diameter of about 2 mm while also providing the flow path between the catheter lumen 42 and the vacuum inlet 380 of the venturi pump 330. When the entrance 362 is sealed around the catheter 40 and the exit 364 is sealed around the inner elongated member 44, the passageway can create a sealed flow path between the lumen 42 of the catheter 40 and the side port 374. The flow path between the side port 374 and vacuum inlet 380 of the venturi pump 330 can also be sealed. The flow path from the catheter lumen 42 to the vacuum inlet 380 can thereby be sealed.

An RHV as known in the art, a variation thereof, or a system, assembly, or device having correlating functionality can serve as the housing 361, side port 374, entrance 362, and exit 364. In reference to FIG. 3, for the sake of readability, the housing, 361, side port 374, entrance 362, and exit 364 are collectively be referred to as the RHV

The flow regulator 324 can be a detachable aspiration control valve and interface such as described in U. S. Patent Application Number 16/400,221. The flow regulator 324 can be conveniently positioned and configured to be manipulated while simultaneously stabilizing the RHV and catheter 40 in one hand and/or retracting an inner elongated member 44 with the other hand.

The assembly can be constructed by attaching the vacuum inlet 380 of the venturi pump 330 to the side port 374 of the RHV and attaching the flow regulator 324 to the supply port 366 of the RHV

The assembly can be made ready for a thrombectomy by closing the flow regulator 324, providing a positive gas pressure supply at the flow regulator 324, and connecting the exhaust port 368 to a waste container. The positive gas pressure supply can be provided by connecting a canister 30 with compressed gas to the flow regulator 324. The exhaust port 368 can be connected to the waste container via a tube 32 or other connector. The RHV can provide structural support for the attached venturi pump 330, flow regulator 324, and canister 30. The assembled canister 30, venturi pump 330, flow regulator 324, and RHV can be sized, weighted, and otherwise designed to be moved and manipulated by hand as needed by a physician during a thrombectomy procedure. The waste container and connection thereto 32 can be sized and positioned as to not inhibit the manipulation of the assembly and canister 30 during the thrombectomy.

As part of a thrombectomy procedure, the catheter 40 can be positioned within a blood vessel 20 of a patient such that the distal end of the catheter 40 is near the clot 10 such as illustrated in FIG. 1, the proximal end of the catheter 40 can be sealed at the entrance 362 of the RHV, and the inner elongated member 44 can be extended within the lumen 42 of the catheter 40 and through the RHV

Once the catheter 40, inner elongated member 44, air canister 30, flow regulator 324, RHV, and venturi pump 330, and discharge connection 32 are configured as described, the flow regulator 324 can be opened. Once the flow regulator 324 is open, gas from the canister 30 can flow through into the supply port 366 and out the exhaust port 368 of the venturi pump 330. The flow can generate suction at the vacuum inlet 380 of the venturi pump. Suction at the vacuum inlet 380 can be transferred to the lumen 42 of the catheter 40 via a flow path in the RHV housing 361 between the entrance 362 and the side port 366. The suction through the catheter lumen 42 can be sufficient to aspirate blood within the blood vessel 20, and/or the suction through the catheter lumen 42 can be sufficient to secure the clot 10 to the distal end of the catheter 40.

The flow regulator 324 can be manipulated by the physician during a thrombectomy to adjust suction through the lumen 42 of the catheter. The flow regulator 324 can include a valve that can be manipulated to adjust the velocity of gas flow through a venturi channel in the venturi pump 330. Because suction at the vacuum inlet 380 of the venturi pump is a function of flow rate through the venturi channel, adjusting the velocity of gas flow through the venturi channel thereby adjusts suction provided at the vacuum inlet 380 of the venturi pump 330. Because the vacuum inlet 380 is in fluidic communication with the entrance 362 of the RHV to which the catheter 40 can attach, adjusting the flow regulator 424 can thereby adjust the suction through the catheter 40.

FIGs. 4A and 4B illustrated venturi pumps that can be integrated into an aspiration device 160, 260 as illustrated in FIGs. 1 and 2 or attached as part of an assembly 360 as illustrated in FIG. 3. There are several known venturi pumps that can be used for the purposes described herein as would be understood by a person of ordinary skill in the art, and therefore the pumps 430a, 430b illustrated and described herein are not intended to encompass all contemplated venturi pump configurations.

FIG. 4A is a cross-sectional illustration of a venturi pump 430a having a supply port 432a, a vacuum inlet 436a, an exhaust port 434a, and a constricted flow venturi channel 474a. During operation, gas and/or liquid flows in the directions indicated by the arrows. As gas or liquid originating from the supply port 432a compresses to pass through the venturi channel 474a, the gas or liquid accelerates such that the gas or liquid exiting the channel 474a has a higher velocity than when it entered the preceding chamber. The increased velocity creates suction at the vacuum inlet 436a due to the Venturi effect. Suction at the vacuum inlet 436a can be increased or decreased by adjusting the velocity of the gas or liquid exiting the channel 474a. The velocity through the channel 474a can be adjusted by adjusting pressure at the supply port 432a. Gas, liquid, and other materials can exit the pump 430a at the exhaust port 434a.

FIG. 4B is a three-dimensional perspective view illustration of a venturi pump 430b having a supply port 432b, vacuum inlet 436b, and exhaust port 434b oriented similarly as the venturi pump 330 illustrated in FIG. 3.

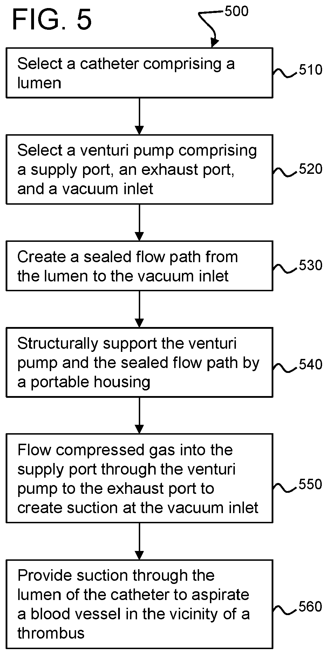

FIG. 5 is a flow diagram including method steps for providing suction through a lumen of a catheter as part of an intravascular treatment. The method steps can be implemented by an example system, device, or assembly as described herein or by a means that would otherwise be known to one of ordinary skill in the art. Referring to the method 500 outlined in FIG. 5, in step 510 a catheter can be selected. The catheter can be a Guide Catheter, catheter suitable for ADAPT, or other catheter that can provide suction to a treatment site. In step 520, a venturi pump having a supply port, exhaust port, and vacuum inlet can be provided. The venturi pump can be a device that can be part of an assembly or integrated with other components or devices. In step 530, a sealed flow path can be created from the lumen of the catheter to the vacuum inlet of the venturi pump. In step 540, the venturi pump and the sealed flow path can be structurally supported by a portable housing. The venturi pump and the sealed flow path can be structurally supported by the portable housing by virtue of being integral to the portable housing and/or rigidly attached to the portable housing. In step 550, compressed gas can be flowed into the supply port, through the venturi pump, and out the exhaust port to create suction at the vacuum inlet. The venturi pump can include a venturi channel that generated suction at the vacuum inlet due to the Venturi effect. In step 560, suction can be provided through the lumen of the catheter to aspirate a blood vessel in the vicinity of a thrombus. The suction can facilitate extraction of the thrombus during a thrombectomy.

The descriptions contained herein are examples of embodiments of the invention and are not intended in any way to limit the scope of the invention. As described herein, the invention contemplates many variations and modifications of the systems, assemblies, devices, and methods for utilizing a venturi pump during an intravascular treatment.

As an alternative to the canister 30, it is contemplated that the compressed gas source can be provided by a central source within a facility (e.g. a tank supplying compressed gas throughout a hospital) and provided to the aspiration control device 260 via tubing connecting to the central source. Using the canister 30 can provide ease of manipulation of the assembly by having one fewer connection to a larger facility, and a canister 30 can be used in facilities that lack a centralized compressed gas source. Using the central source within the facility can provide a reliable, continuous supply of compressed air during a procedure and can eliminate the need to restock or refill individual canisters.

As an alternative to the waste container 34 and tubing 32, it is contemplated that a waste canister can be rigidly connected to an aspiration control device or assembly 160, 260, 360 such that the waste canister is structurally supported by the device or assembly (e.g. supported by a portable housing 161, 261, 361). The waste canister can be positioned such that gas exhaust and waste 22 can exit the aspiration the device or assembly 160, 260, 360 in such a way that is non-obstructive to a physician manipulating the device or assembly 160, 260, 360 during a thrombectomy.

Additional variations and modifications of the systems, assemblies, and devices include integrating components to form a single device, connecting components to form an assembly, utilizing various configurations of a flow regulator and control valve, using various configurations of a venturi pump, adapting or modifying an RHV in various ways, incorporating fittings or adapters to "directly" connect components, etc. These modifications would be apparent to those having ordinary skill in the art to which this invention relates and are intended to be within the scope of the claims which follow.

Any or all of the methods described herein may be methods that are not methods for treatment of the human or animal body by surgery, therapy or diagnosis. For example, the methods may take place in vitro and/or ex vivo e.g. in a laboratory, for testing, etc. The methods may be methods of preparing for performing a thrombectomy.

### Embodiments of the invention:

1. A method for performing a thrombectomy, the method comprising:
   selecting a catheter comprising a lumen;
   selecting a venturi pump comprising a supply port an exhaust port, and a vacuum inlet;
   creating a sealed flow path from the lumen of the catheter to the vacuum inlet of the venturi pump;
   structurally supporting the venturi pump and the sealed flow path by a portable housing;
   flowing compressed gas from the supply port to the exhaust port of the venturi pump, thereby creating suction at the vacuum inlet and through the sealed flow path; and
   providing the suction through the lumen of the catheter to aspirate a blood vessel in the vicinity of a thrombus.
2. The method of embodiment 1 further comprising:
   continuously flowing compressed gas from the supply port to the exhaust port of the venturi pump thereby securing the thrombus to the catheter while the catheter is retracted proximally through the blood vessel.
3. The method of embodiment 1 further comprising:
   extracting the thrombus from the vessel with the catheter using A Direct Aspiration first-Pass Technique.
4. The method of embodiment 1 further comprising:
   inserting a proximal end of the catheter through an entrance in the portable housing;
   extending an Intermediate Catheter from within the lumen of the catheter, out of the proximal end of the catheter, through the portable housing, and out an exit in the portable housing;
   sealing an outer diameter of the catheter at the entrance of the portable housing; and
   sealing an outer diameter of the Intermediate Catheter at the exit in the portable housing.
5. The method of embodiment 1 further comprising:
   holding the portable housing by hand while providing the suction through the lumen of the catheter to aspirate the blood vessel in the vicinity of the thrombus.
6. The method of embodiment 1 further comprising:
   manipulating the Intermediate Catheter while providing the suction through the lumen of the catheter to aspirate the blood vessel in the vicinity of the thrombus.

## Claims

1. A suction device comprising:
an entrance comprising a seal movable to receive a catheter suitable for navigating a blood vessel in a patient and hemostatically seal around an outer diameter of the catheter;
a venturi pump comprising a supply port receiving a positive pressure gas supply, an exhaust port, and a vacuum inlet, the vacuum inlet in fluidic communication with the entrance of the suction device; and
a portable housing providing structural support for the entrance and the venturi pump.

2. The suction device of claim 1 further comprising:
a flow regulator valve in communication with the venturi pump.

3. The suction device of claim 1 further comprising:
a compressed gas canister providing the positive gas pressure supply, the canister detachably attached to the supply port, and structurally supported by, the portable housing; and
a discharge container positioned to receive discharge from the exhaust port of the venturi pump.

4. The suction device of claim 1 further comprising:
an exit comprising a seal movable to receive an inner elongated member positioned within the catheter and hemostatically seal around an outer diameter of the inner elongated member, the exit structurally supported by the portable housing; and
a passageway within the portable housing sized to allow the inner elongated member to extend between the entrance and the exit and sized to provide a flow path between the entrance and the vacuum inlet of the venturi pump when the inner elongated member is linearly extended between the entrance and the exit.

5. The suction device of claim 4,
wherein the catheter is a Guide Catheter, and
wherein the inner elongated member is an Intermediate Catheter having an outer diameter of approximately 2 mm.

6. The suction device of claim 4,
wherein the entrance further comprises a first locking mechanism movable to grip the catheter to inhibit longitudinal and rotational movement of the catheter in relation to the housing of the suction device, and
wherein the exit further comprises a second locking mechanism movable to grip the inner elongated member to inhibit longitudinal and rotational movement of the inner elongated member in relation to the housing of the suction device.

7. The suction device of claim 1 further comprising:
the catheter,
wherein the catheter is suitable for capturing a clot as part of A Direct Aspiration first-Pass Technique.

8. A handheld endovascular treatment assembly comprising:
a first opening comprising a seal movable to receive a catheter and hemostatically seal around an outer diameter of the catheter;
a second opening;
a third opening; and
a venturi channel in fluidic communication with the first opening, second opening, and third opening,
wherein the venturi channel provides a restricted flow path from the second opening to the third opening and a vacuum inlet at the third opening, and
wherein the handheld endovascular treatment assembly is sized to be handheld during an endovascular treatment.

9. The handheld endovascular treatment assembly of claim 8 further comprising:
a flow regulator valve in communication with the second opening.

10. The handheld endovascular treatment assembly of claim 8 further comprising:
a compressed gas canister detachably attached and structurally supported within the handheld endovascular treatment assembly.

11. The handheld endovascular treatment assembly of claim 8 further comprising:
a fourth opening comprising a seal movable to receive an inner elongated member positioned within the catheter, the seal movable to hemostatically seal around an outer diameter of the inner elongated member; and
a passageway sized to allow the inner elongated member to extend between the first opening and the fourth opening and sized to provide a flow path from the first opening to the third opening.

12. The handheld endovascular treatment device of claim 11, wherein the inner elongated member has an outer diameter of approximately 2 mm.

13. The handheld endovascular treatment device of claim 11,
wherein the first opening further comprises a first locking mechanism movable to grip the catheter to inhibit longitudinal and rotational movement of the catheter in relation to the handheld endovasculature treatment assembly, and
wherein the fourth opening further comprises a second locking mechanism movable to grip the inner elongated member to inhibit longitudinal and rotational movement of the inner elongated member in relation to the handheld endovasculature treatment assembly.

14. A system comprising the handheld endovascular treatment assembly of claim 9 and the catheter,
wherein the catheter is suitable for capturing a clot as part of A Direct Aspiration first-Pass Technique.

15. A method of preparing for performing a thrombectomy, the method comprising:
selecting a catheter comprising a lumen;
selecting a venturi pump comprising a supply port an exhaust port, and a vacuum inlet;
creating a sealed flow path from the lumen of the catheter to the vacuum inlet of the venturi pump;
structurally supporting the venturi pump and the sealed flow path by a portable housing;
flowing compressed gas from the supply port to the exhaust port of the venturi pump, thereby creating suction at the vacuum inlet and through the sealed flow path.
